## Europäisches Patentamt

## European Patent Office

## Office européen des brevets

(19)

(11) Veröffentlichungsnummer: **0 160 909 B1**

(12) **EUROPÄISCHE PATENTSCHRIFT**

(45) Veröffentlichungstag der Patentschrift:
09.03.88

(51) Int. Cl.⁴: **C 07 C 139/02, C 07 C 143/70**

(21) Anmeldenummer: **85105170.6**

(22) Anmeldetag: **27.04.85**

(54) **Verfahren zur Chlorierung und Sulfochlorierung von organischen Verbindungen.**

(30) Priorität: **05.05.84 DE 3416668**

(43) Veröffentlichungstag der Anmeldung:
**13.11.85 Patentblatt 85/46**

(45) Bekanntmachung des Hinweises auf die Patenterteilung:
**09.03.88 Patentblatt 88/10**

(84) Benannte Vertragsstaaten:
**AT BE CH DE FR GB IT LI LU NL SE**

(56) Entgegenhaltungen:
**DE - A - 3 238 741**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Carduck, Franz-Josef, Dr., Landstrasse 19, D-5657 Haan (DE)**
Erfinder: **Wüst, Willi, Dr., Fasanenring 32, D-4030 Ratingen-Hösel (DE)**
Erfinder: **Harth, Hubert, Dr., Buchenstrasse 31, D-4000 Düsseldorf 13 (DE)**
Erfinder: **Liebs, Harald, Solinger Strasse 30, D-5090 Leverkusen 1 (DE)**

ACTORUM AG

## Beschreibung

Die Erfindung betrifft ein verbessertes Verfahren zur Umsetzung von Chlor sowie Gemischen aus Chlor und Schwefeldioxid mit flüssigen oder gelösten organischen Komponenten. Das Verfahren ist insbesondere dann von Vorteil, wenn die zu behandelnde Flüssigphase unter Reaktionsbedingungen eine erhöhte Viskosität aufweist oder annimmt. Das Verfahren eignet sich insbesondere zur Chlorierung und Sulfochlorierung von Fettrohstoffen, insbesondere von Fettsäuren und/oder deren Estern mit ein- und/oder mehrwertigen Alkoholen. Der Begriff der Chlorierung umfasst im Rahmen der erfindungsgemässen Lehre sowohl die Addition von Chlor an olefinische Doppelbindungen des Ausgangsmaterials als auch die gegebenenfalls durch UV-Licht angeregte Substitutionsreaktion unter Bildung von HCl. Der Begriff der Sulfochlorierung erfasst in üblicher Weise die gegebenenfalls durch UV-Licht angeregte Reaktion von $SO_2/Cl_2$-Gemischen mit aliphatischen Kettenbestandteilen unter Bildung der $SO_2Cl$-Gruppe und Emission von HCl.

Die Chlorierung und Sulfochlorierung von Fett-Rohstoffen, insbesondere Fettsäuren und/oder Fettsäureestern führt bekanntlich zu interessanten Fettungsmitteln für Leder und Pelze. So sind aus der DE-PS 2245077 solche Fettungsmittel auf Basis von sulfonierten Chlorierungsprodukten von natürlichen oder synthetischen höheren Fettsäuren oder Fettsäureestern in Form ihrer Alkali-, Ammonium- oder Aminsalze bekannt, die dadurch gekennzeichnet sind, dass sie aus solchen sulfonierten Chlorierungsprodukten bestehen, die durch Chlorieren von höheren Fettsäuren oder von Estern höherer Fettsäuren der Kettenlängen $C_8$–$C_{24}$ bis zu einem Chlorgehalt von 20 bis 45 Gewichtsprozent erhalten worden sind, wobei die Chlorierungsprodukte im wesentlichen keine olefinischen Doppelbindungen mehr enthalten und die nachfolgende Sulfonierung mit $SO_3$ bis zu einem Gehalt von 40 bis 100 Molprozent $SO_3$, bezogen auf Chlorierungsprodukt, durchgeführt worden ist.

Entsprechende Fettungsmittel beschreibt weiterhin die DE-OS 3018176, wobei diese Lehre dadurch gekennzeichnet ist, dass die Fettungsmittel im wesentlichen aus solchen sulfonierten Chlorierungsprodukten bestehen, die durch Sulfochlorieren von höheren Fettsäuren oder von Estern höherer Fettsäuren der Kettenlängen $C_8$–$C_{24}$ mittels Chlor und $SO_2$ gegebenenfalls unter UV-Bestrahlung bei 20 bis 90°C bis zu einem Gehalt an gebundenen Chlor von 5 bis 30 Gewichtsprozent und einem Gehalt an $SO_2Cl$-Gruppen von 1 bis 20 Gewichtsprozent hergestellt worden sind, wobei das Verhältnis von Chloratomen zu $SO_2Cl$-Gruppen im Bereich von 0,7:1 bis 70:1 liegt und eine nachfolgende Verseifung dieser Gruppen vorgenommen worden ist. Eine Verbesserung wird in der DE-OS 3238741.5 geschildert, wobei jetzt im Rahmen der Herstellung der sulfonierten Chlorierungsprodukte von höheren Fettsäure- oder Fettsäureester-Gemischen ausgegangen wird, die ungesättigte Anteile enthalten. In diesem Fall wird zunächst eine Chlorierung zur Absättigung der Doppelbindungen und danach eine Sulfochlorierung mit Chlor und $SO_2$ durchgeführt. Die nachfolgende Verseifung führt zu den gewünschten Fettungsmitteln.

Unter den Reaktionsbedingungen der Chlorierung/Sulfochlorierung liegen Chlor und $SO_2$ gasförmig vor. Um mit dem organischen Reaktionspartner reagieren zu können, müssen diese gasförmigen Komponenten in der flüssigen Phase gelöst werden. Üblicherweise leitet man hierzu die Gase über Gasverteiler von unten in eine Flüssigkeitssäule ein oder aber man lässt gasförmige und flüssige Reaktanten im Gegenstrom zueinander durch Austauschvorrichtungen, beispielsweise Füllkörperkolonnen, laufen. Es zeigt sich jedoch dabei, dass die Viskosität der organischen Flüssigphase mit zunehmendem Reaktionsablauf zunehmend ansteigt. Gleichzeitig wird der Stoffaustausch zwischen flüssiger Phase und Gasphase zunehmend gehemmt. So beobachtet man beispielsweise in einer Säule des flüssigen Reaktanten, in die unten die gasförmigen Reaktanten eingespeist werden, mit zunehmender Viskosität der Flüssigphase immer grössere Blasen, die sehr rasch aufsteigen. Die dem Reaktor zugeführten gasförmigen Komponenten verlassen im zunehmenden Ausmass den Reaktor im unreagierten Zustand. Die durch die Stoffübergangsgeschwindigkeit der Gase in die Flüssigphase beeinflusste Umsetzungsgeschwindigkeit sinkt. Bei simultaner Absorption und Reaktion mehrerer Gase beeinflusst die unterschiedliche Löslichkeit der Gase in der Flüssigphase den Reaktionsverlauf.

Die Lehre der Erfindung geht von der überraschenden Feststellung aus, dass trotz der zu beobachtenden zunehmend im Reaktionsverlauf verschwerten Vermischbarkeit von Gas- und Flüssigphasen durch zusätzliche Einwirkung mechanischer Kräfte eine Homogenisierung des Reaktionsgemisches insbesondere in den Stadien des Reaktionsablaufes erreicht werden kann, in denen an sich das optische Erscheinungsbild des Reaktantengemisches gegen gute Vermischbarkeit spricht.

Gegenstand der Erfindung ist dementsprechend ein Verfahren zur Chlorierung und Sulfochlorierung von unter Reaktionsbedingungen flüssigen Fett-Rohstoffen, insbesondere Fettsäuren und/oder deren Estern, durch Umsetzung mit Chlor und $SO_2$, bei dem man das flüssige Einsatzmaterial in Kreislaufführung durch eine Reaktionszone führt, in der es mit in den Kreislauf eingespeistem Chlor und $SO_2$ zur Umsetzung gebracht wird, wobei das neue Verfahren dadurch gekennzeichnet ist, dass das Chlor und $SO_2$ im Gleichstrom mit dem flüssigen Gut eingespeist und zusammen mit diesem beim ober vor dem Eintritt in die Reaktionszone durch einen Intensivmischer geführt werden, wobei die Menge des eingespeisten Chlors und $SO_2$ so bemessen wird, dass das Reaktionsgemisch die Reaktionszone als praktisch homogene Flüssigphase durchläuft.

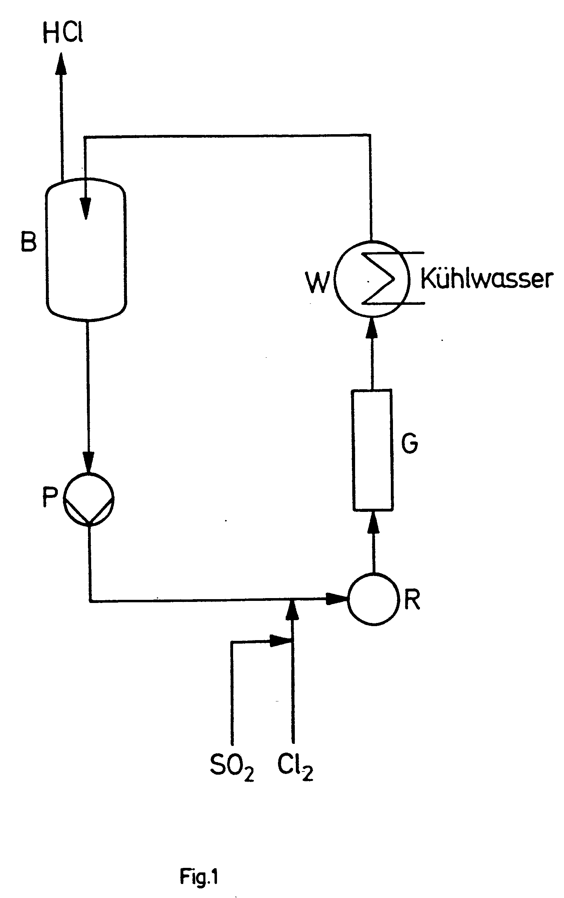

Das Prinzip des erfindungsgemässen Verfahrens wird aus dem Fliessschema der beigefügten Figur 1 ersichtlich: Die zu chlorierende/sulfochlorierende Flüssigkeit wird aus dem Behälter B mit der Pumpe P der Mischeinrichtung R zugeführt. Kurz vor Eintritt – oder beim Eintritt – in R wird in die umlaufende Flüssigkeit zweckmässig durch ein Koaxialrohr die gasförmige Reaktante bzw. das entsprechende Reaktantengemisch eingeleitet. Als Mischzone können in den Flüssigkeitskreislauf eingefügte statische und/oder bewegte Mischelemente eingesetzt werden, wobei bevorzugt mit statischen Mischelementen gearbeitet wird, die durch sich kreuzende Leitelemente im Bereich der Mischzone eine hochturbulente Zick-Zack-Strömung des Gas-Flüssigkeits-Gemisches bewirken.

Durch die intensive mechanische Vermischung von Gas-Flüssig-Phase in der Mischeinrichtung R wird trotz kurzer Verweilzeit des Reaktionsgemisches in dieser Mischeinrichtung die Gasphase vollständig oder praktisch vollständig in der zunehmend viskosen Flüssigphase gelöst. In der bevorzugten Ausführungsform der Erfindung enthält die Flüssigkeit, welche die Mischzone R verlässt, praktisch keine sichtbaren Gasblasen. Sie wird über die Reaktionszone C und anschliessend durch einen Wärmetauscher W in den Behälter B zurückgeführt. Hier gast die gegebenenfalls gebildete HCl aus und kann einem Wäscher zugeführt werden.

Die beschriebene Verfahrensweise lässt zu, dass die Reaktion in einem einphasigen, homogenen Medium stattfindet. Das hat u.a. die folgenden Vorteile:

Die Umsetzungsgeschwindigkeit kann nicht durch den Stoffübergang gas-flüssig limitiert werden, die unterschiedlichen Löslichkeiten von Chlor und $SO_2$ in der Flüssigphase haben damit keinen Einfluss auf den Reaktionsablauf. Chlor und $SO_2$ haben eine einheitliche Verweilzeit in der Reaktionszone, d.h. ein enges Verweilzeitspektrum. Die Verweilzeit von Chlor bzw. $SO_2$ in der Reaktionszone wird nur durch den Flüssigkeitskreislauf bestimmt. Ein Durchschlagen von nicht reagiertem Gas ist nicht möglich. Es können Flüssigphasen mit hoher Viskosität eingesetzt werden, die bisher wegen des Durchschlagens der Gasphase wirtschaftlich der Umsetzung nicht zugeführt werden konnten. Die Reaktion selbst kann bei niedrigeren Temperaturen – und damit entsprechend höheren Viskositäten der Flüssigphase – durchgeführt werden, was beispielsweise hinsichtlich der Selektivität der Sulfochlorierung von Vorteil ist. Die Viskosität beeinflusst damit nicht mehr die Wahl der Reaktionstemperatur. Die Sulfochlorierung kann damit problemlos bei einem Einsatzmolverhältnis von Chlor zu $SO_2$ von 1:1 durchgeführt werden, ohne dass gleichzeitig Kettenchlorierung eintritt oder es zu wesentlichen $SO_2$-Verlusten kommt. Dies ist beispielsweise von Interesse, wenn preisgünstige Fettstoffe mit höherer Jodzahl zum Einsatz kommen. Mit diesen Fettstoffen kann zunächst eine Chloraddition an die olefinische Doppelbindung und anschliessend eine Sulfochlorierung ohne Kettenchlorierung durchgeführt werden. Für denselben Kettenchlorgehalt wird bekanntlich bei der Additionsreaktion nur halbsoviel Chlor verbraucht wie bei der Substitutionsreaktion.

Das neue Verfahren ist insbesondere geeignet für die Chlorierung/Sulfochlorierung von Fettstoffen wie Triglyceriden, Fettsäuren und/oder deren Estern. Beispielsweise sind Talg, Talgfettsäuremethylester, Kokosfettsäuremethylesternachlauf, LT-Nachlaufester und deren Gemische.

Bevorzugt geht die Erfindung von höheren Fettsäuren oder von Estern solcher höherer Fettsäuren der Kettenlängen $C_8$–$C_{24}$, insbesondere $C_{10}$–$C_{20}$, mit Jodzahlen von 10–120 aus. Bevorzugt werden dabei Gemische von Fettsäuren aus natürlich vorkommenden Fetten oder Ölen mit einem Anteil an einfach oder mehrfach ungesättigten Fettsäuren. Beispiele hierfür sind die aus Kokosöl, Sojaöl, Palmkernöl, Baumwollsaatöl, Rüböl, Leinöl, Riciniusöl, Sonnenblumenöl, Olivenöl, Klauenöl, Erdnussöl, Heringstran, Dorschtran, Haifischtran, Waltran, Talgfetten oder Schweineschmalz gewonnenen Fettsäuregemische bzw. deren Ester. Auch die entsprechenden natürlich vorkommenden Fette oder Öle sowie natürlich vorkommende Wachsester, z.B. Spermöl, kommen als Ausgangsmaterial für die Herstellung von Fettungsmitteln der beschriebenen Art in Betracht. Entscheidend ist, dass die Durchführung der Chlorierung und Sulfochlorierung jetzt nicht mehr durch die hohe Viskosität der entsprechenden Flüssigphasen bzw. daraus entstehenden Reaktionsprodukte behindert wird.

Besonders bevorzugt als Ausgangsstoffe für die Herstellung der Fettungsmittel sind synthetisch hergestellte Ester aus Gemischen gesättigter und ungesättigter Fettsäuren der Kettenlängen $C_8$ bis $C_{24}$, vorzugsweise $C_{10}$ bis $C_{20}$, mit Jodzahlen von 10 bis 120, beispielsweise Decancarbonsäure, Palmitinsäure, Stearinsäure, Behensäure, Dodecencarbonsäure, Ölsäure, Linolsäure oder durch Paraffinoxidation hergestellte Carbonsäuren und deren Ester mit einwertigen aliphatischen Alkoholen der Kettenlängen $C_1$ bis $C_4$. Wegen ihrer leichten Zugänglichkeit können auch die aus natürlichen tierischen oder pflanzlichen Fetten, Ölen oder Wachsen hergestellten Umesterungsprodukte mit den niederen einwertigen aliphatischen Alkoholen, insbesondere Methanol, erhaltenen Fettsäureester bevorzugt sein. Andere zur Esterbildung geeignete Alkohole sind: mehrwertige aliphatische Alkohole der Kettenlängen $C_2$ bis $C_6$ wie Ethylenglykol, 1,2-Propylenglykol, Glycerin, Pentaerythrit oder Sorbit oder auch höhere Alkohole der Kettenlängen $C_8$ bis $C_{24}$, wie Decylalkohol oder Oleylalkohol.

In einer bevorzugten Ausführungsform der Erfindung wird die Reaktion in Abwesenheit von viskositätserniedrigenden Verdünnungsmitteln durchgeführt. Bekanntlich war zur Lösung des Viskositätsproblems im hier betroffenen Arbeitsgebiet auch schon vorgeschlagen, durch Mitverwendung inerter viskositätserniedrigender Verdünnungsmittel einen verbesserten Stoffaustausch zwischen Gas- und Flüssigphase zu erreichen.

Als Mischelemente – vergleiche Abbildung 1R – sind beispielsweise kommerziell erhältliche Rotor-Stator-Maschinen geeignet. In diesen Vorrichtungen erzeugen die schnell laufenden Rotoren eine mechanische Scherwirkung zwischen Rotor und Stator. Durch diese Scherwirkung wird das Gas während der kurzen Verweilzeit in der Mischeinrichtung vollständig gelöst. Die homogene bzw. praktisch homogene Flüssigphase, die die Rotor-Stator-Maschine verlässt, wird der gewöhnlich mit einer UV-Lampe bestrahlten Reaktionszone G zugeführt.

In einer bevorzugten Ausführungsform der Erfindung wird mit statischen Mischelementen gearbeitet. Auch hierbei handelt es sich um handelsübliche Hilfsmittel. Geeignet sind beispielsweise Einbaukörper aus gewellten und vielfach abgeknickten, sich kreuzenden Streckmetallbändern. In einem solchen System sich kreuzender Kanäle ist eine hochturbulente Zick-Zack-Strömung des Gemisches von Flüssigphase und gasförmigem Reaktanten gewährleistet. Hierdurch wird die intensive Vermischung und letztlich damit die Homogenisierung der Reaktionsphase erreicht. Zur einschlägigen Literatur wird beispielsweise verwiesen auf W. Tauscher, Das breite Anwendungsspektrum des statischen Mischers, Chemische Produktion (1977) 10, 10–14. Geeignet sind beispielsweise entsprechende statische Mischelemente der Firma Gebrüder Sulzer AG, Winterthur, Schweiz, die beispielsweise unter der Handelsbezeichnung «SMV» vertrieben werden.

Insbesondere für das Arbeiten mit solchen in das Fliessrohr eingebauten statischen Mischelementen gelten die folgenden bevorzugten Verfahrensangaben: Fliessgeschwindigkeiten der Flüssigphase in der statischen Mischzone – bezogen auf das leere Rohr – im Bereich von 0,1 bis 5 m/s, vorzugsweise im Bereiche von 0,25 bis 2,5 m/s. Bevorzugtes Verhältnis von Länge zu Durchmesser der Mischstrecke im Bereiche von 2:1 bis 20:1. Mit vergleichsweise kurzen Mischstrecken, die etwa im Bereich von 10 bis 50 cm liegen können, wird eine Intensivvermischung im erfindungsgemässen Sinne erzielt. Das Verhältnis der zugeführten Gasmenge zur umlaufenden Flüssigkeitsmenge liegt für die beiden Reaktionsgase Chlor und $SO_2$ jeweils im allgemeinen im Bereich von 0,05 bis 500 Gewichtsteile Gas/1000 Gewichtsteile Flüssigkeit, insbesondere im Bereich von 0,1 bis 100 Gewichtsteile Gas und vorzugsweise im Bereich von 0,3 bis 30 Gewichtsteile Gas – jeweils bezogen auf 1000 Gewichtsteile Flüssigkeit.

Chlor und Schwefeldioxid können als gasförmige Reaktanten aber auch unter Druck als Flüssigkeiten in den umlaufenden Strom des Flüssigreaktanten eindosiert werden, wobei sie – abhängig von Druck und Temperatur an der Einleitstelle – entweder verdampfen oder als Flüssigkeit dispergiert und gelöst werden. Der Druck an der Einleitstelle von Chlor und/oder Schwefeldioxid liegt bevorzugt im Bereich von 0,2 bis 80 bar, insbesondere im Bereich von 0,2 bis 25 bar und zweckmässigerweise im Bereich von 0,5 bis 5 bar.

Wird erfindungsgemäss mehrstufig derart gearbeitet, dass in einer ersten Reaktionsstufe die Addition von Chlor an vorhandene olefinische Doppelbindungen erfolgt und nachfolgend die Stufe der Sulfochlorierung stattfindet, kann es bevorzugt sein, die Chloraddition im Temperaturbereich von etwa 50 bis 70°C und die Sulfochlorierung bei Temperaturen von etwa 30 bis 50°C durchzuführen.

Die Dauer der Reaktion beträgt im allgemeinen etwa 2 bis 10 Stunden. Nach dieser Zeit sind beispielsweise 5 bis 30 Gewichtsprozent Chlor und 1 bis 20 Gewichtsprozent $SO_2Cl$-Gruppen angelagert worden. Das Verhältnis von Chloratomen zu $SO_2Cl$-Gruppen liegt bevorzugt im Bereich von etwa 0,7:1 bis 70:1, insbesondere beträgt das Verhältnis 2:1 bis 20:1, zweckmässigerweise 3:1 bis 7:1.

Durch nachfolgende Verseifung beispielsweise mit wässriger etwa 30%iger Natrium-Kaliumhydroxidlösung bei etwa 70°C erhält man flüssige hochkonzentrierte wasseremulgierbare Produkte mit ausgezeichneter Oxidations-, Licht- und Säurestabilität, die sich beispielsweise für die Lederfettung hervorragend eignen, so wie es in der DE-OS 3238741.5 im einzelnen beschrieben ist.

In den nachfolgenden Beispielen wird sowohl die Addition von Chlor an vorliegende olefinische Doppelbindungen als auch die Sulfochlorierung im Molverhältnis 1:1 und gegebenenfalls eine nachfolgende Substitution durch Chlor bei höheren Viskositäten beschrieben.

Beispiel 1

841 kg Talgfettsäuremethylester von 20°C (JZ = 52) werden in den in Abbildung 1 gezeigten Behälter B gefüllt und mittels der Pumpe P mit einem Durchsatz von 15 m³/h umgepumpt. Ohne in der Reaktionszone G die UV-Lampe zu zünden, werden 54 kg Chlor/h eingeleitet, wobei die Produkttemperatur bis auf 61°C ansteigt. Nachdem der Chlorgehalt des Produktes 14 Gewichtsprozent erreicht hat, wird auf 40°C abgekühlt und in der Reaktionszone G die UV-Lampe eingeschaltet. Es werden jetzt 20 kg/h Chlor und 18 kg/h $SO_2$ eingeleitet, bis der $SO_2Cl$-Gehalt des Reaktionsproduktes 15 Gewichtsprozent beträgt. Der Kettenchlorgehalt hat sich nicht geändert. Nun wird der $SO_2$-Zustrom abgestellt, und es wird unter denselben Betriebsbedingungen mit 20 kg $Cl_2$/h bis zu einem Kettenchlorgehalt von 18% chloriert. Es werden 1250 kg Talgfettsäuremethylester-Sulfochlorid erhalten.

Während der Reaktion steigt die Viskosität der Flüssigphase auf 300 mPas bei 40°C an. Trotzdem sind durch den in der Mischzone R vorgesehenen Rotor-Stator-Mischer bei laufender Mischvorrichtung hinter dieser keinerlei Gasblasen in der umlaufenden Flüssigphase zu erkennen. Wird jedoch die Rotor-Stator-Maschine ausgestellt, so beobachtet man, wie das Gas in bis zu faustgrossen Blasen rasch nach oben steigt ohne sich zu lösen.

Das Abgas aus dem Behälter B ist von dem durchschlagenden Chlorgas gefärbt.

## Beispiel 2

Es wird wiederum in einem Reaktionskreislauf gemäss Abbildung 1 gearbeitet, als Mischeinheit R liegt jetzt jedoch ein statischer Mischer vor. Hierbei handelt es sich um vier Elemente des von der Firma Gebrüder Sulzer AG, Winterthur, Schweiz unter der Handelsbezeichnung «SMV 8» vertriebenen Typs. Die Gesamtlänge der Mischstrecke beträgt 20 cm, der Durchmesser 5 cm, das Verhältnis von Länge zu Durchmesser somit 4:1.

432 kg Talgfettsäuremethylester/TME) von 20 °C (JZ = 52) werden in den Behälter B gefüllt und mit einem Durchsatz von 9 m³/h umgepumpt. Es werden 98 kg/h Chlor eingeleitet, wobei die Temperatur der Flüssigphase auf 58 °C ansteigt. Nachdem der Chlorgehalt des Produktes 18 Gewichtsprozent erreicht hat, wird auf 41 °C abgekühlt, die UV-Lampe in der Reaktionszone G wird eingeschaltet, und es werden 14,2 kg/h Cl₂ und 12,8 kg/h SO₂ eingeleitet, bis der SO₂Cl-Gehalt 16,2 Gewichtsprozent beträgt. Der Kettenchlorgehalt hat sich nicht geändert. Es werden 652 kg Chlortalgfettsäuremethylester-Sulfochlorid erhalten.

Unter den Verfahrensbedingungen sind in der umgepumpten Flüssigphase nach Durchlaufen der Mischzone R praktisch keine gasförmigen Reaktanten visuell feststellbar.

## Beispiel 3

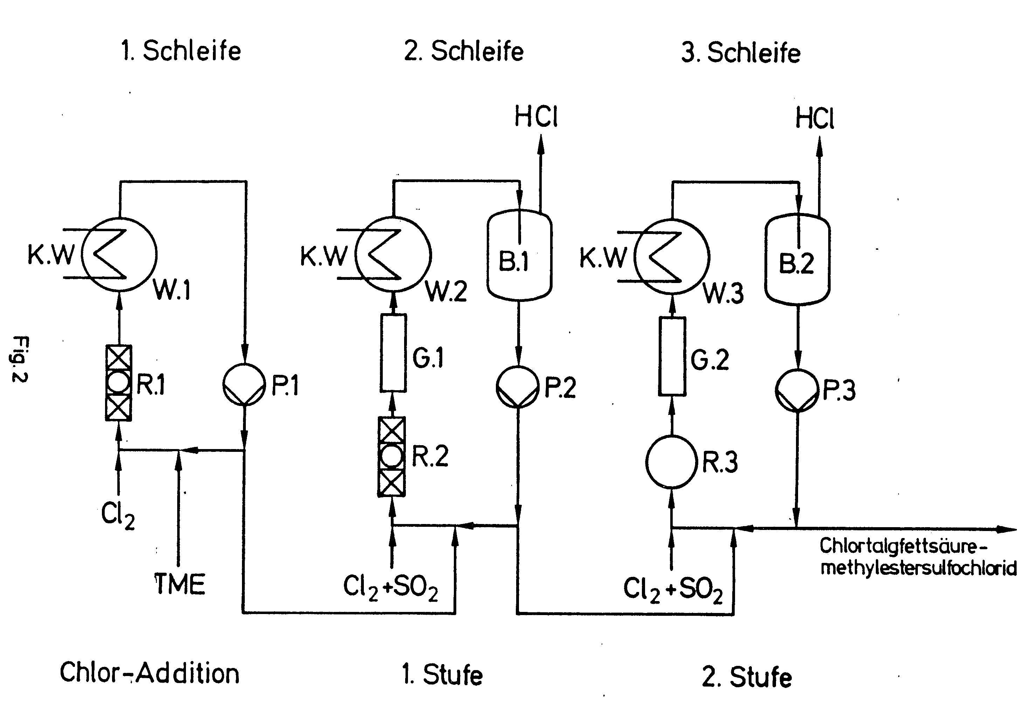

Im nachfolgenden Beispiel wird die kontinuierliche Verfahrensführung unter Bezugnahme auf die beigefügte Abbildung 2 beschrieben.

In der ersten Reaktionsschleife wird die Addition von Chlor an die olefinischen Doppelbindungen des eingesetzten Ausgangsmaterials durchgeführt. Die Reaktionsschleife besteht aus der Umlaufpumpe P1, der statischen Mischstrecke R1 und dem Wärmeaustauscher W1. In ihr werden 5 m³/h Flüssigphase von 60 °C umgepumpt. Es werden 260 kg/h Talgfettsäuremethylester und 36,4 kg/h Chlor kontinuierlich zudosiert und 296,4 kg/h Chlortalgfettsäuremethylester abgezogen, die der zweiten Reaktionsschleife zugeführt werden. Hier finden simultan die Sulfochlorierung und die Chlorierung statt. In der 1. Stufe der Sulfochlorierung/Chlorierung, bestehend aus der Umlaufpumpe P2, der statischen Mischstrecke R2, und der UV-Lampe G1 dem Wärmetauscher W2 und dem Abscheider B1 werden 9 m³/h Flüssigphase bei 40 °C umgepumpt. Es werden 51,9 kg/h Cl₂ sowie 20,8 kg/h SO₂ kontinuierlich zudosiert und 342,4 kg/h Chlortalgfettsäuremethylestersulfochlorid in Richtung der dritten Schleife abgezogen. Gleichzeitig verlassen 26,7 kg/h HCl-Gas den Abscheider B1. In der 2. Stufe der Sulfochlorierung/Chlorierung ist als Mischorgan eine Rotor-Stator-Maschine (R3) eingebaut. Es werden 15 m³/h Flüssigphase von 40 °C umgepumpt und 46,8 kg/h Cl₂ sowie 18,2 kg SO₂/h kontinuierlich zudosiert. 24,1 kg/h HCl-Gas werden im Abscheider B₂ abgetrennt. Es werden 383,3 kg/h Chlortalgfettsäuremethylestersulfochlorid mit 16,7 Gewichtsprozent Kettenchlor und 15,8 Gewichtsprozent SO₂Cl erhalten.

## Patentansprüche

1. Verfahren zur Chlorierung und Sulfochlorierung von unter Reaktionsbedingungen flüssigen Fett-Rohstoffen, insbesondere Fettsäuren und/oder deren Estern, durch Umsetzung mit Chlor und SO₂, bei dem man das flüssige Einsatzmaterial in Kreislaufführung durch eine Reaktionszone führt, in der es mit in den Kreislauf eingespeistem Chlor und SO₂ zur Umsetzung gebracht wird, dadurch gekennzeichnet, dass das Chlor und SO₂ im Gleichstrom mit dem flüssigen Gut eingespeist und zusammen mit diesem beim oder vor dem Eintritt in die Reaktionszone durch einen Intensivmischer geführt werden, wobei die Menge des eingespeisten Chlors und SO₂ so bemessen wird, dass das Reaktionsgemisch die Reaktionszone als praktisch homogene Flüssigphase durchläuft.

2. Verfahren nach Anspruch 1, dadurch gekennzeichnet, dass man mit einem olefinisch ungesättigte Anteile enthaltenden Ausgangsmaterial und dabei vorzugsweise mehrstufig derart arbeitet, dass zunächst Chlor an die Doppelbindungen angelagert und nachfolgend sulfochloriert wird, wobei gewünschtenfalls in einer der Sulfochlorierung vorgängigen oder nachfolgenden Reaktionsstufe oder während der Sulfochlorierung der Kettenchlorgehalt des Einsatzgutes durch Substitutionsreaktion erhöht wird.

3. Verfahren nach Ansprüchen 1 und 2, dadurch gekennzeichnet, dass man Ester von Fettsäuren der Kettenlängen C₈–C₂₄ mit ein- und/oder mehrwertigen Alkoholen dem Verfahren unterwirft, wobei bevorzugt Ester von Fettsäuren bzw. Fettsäuregemischen natürlichen Ursprungs eingesetzt werden.

4. Verfahren nach Ansprüchen 1 bis 3, dadurch gekennzeichnet, dass man in Abwesenheit von viskositätserniedrigenden Verdünnungsmitteln arbeitet.

5. Verfahren nach Ansprüchen 1 bis 4, dadurch gekennzeichnet, dass man die intensive Vermischung von Gas- und Flüssigphase durch an sich bekannte in den Flüssigkeitskreislauf eingefügte statische und/oder bewegte Mischelemente bewirkt, wobei bevorzugt mit statischen Mischelementen gearbeitet wird, die durch sich kreuzende Leitelemente im Bereich der Mischzone eine hochturbulente Zick-Zack-Strömung des Gas-Flüssigkeitsgemisches auslösen.

6. Verfahren nach Ansprüchen 1 bis 5, dadurch gekennzeichnet, dass mit Fliessgeschwindigkeiten der Flüssigphase in der statischen Mischzone – bezogen auf das leere Rohr – von 0,1 bis 5 m/sec., insbesondere im Bereich von 0,25 bis 2,5 m/sec. gearbeitet wird, wobei bevorzugt das Verhältnis von Länge zu Durchmesser der Mischstrecke im Bereich von 2:1 bis 20:1 liegt.

7. Verfahren nach Ansprüchen 1 bis 6, dadurch gekennzeichnet, dass das Verhältnis der zugeführten Gasmenge zur umlaufenden Flüssigkeitsmenge für beide gasförmigen Reaktanten im Bereich von 0,05 bis 500 kg Gas/1000 kg Flüssigkeit, insbesondere im Bereich von 0,1 bis 100 kg Gas/1000 kg Flüssigkeit liegt.

8. Verfahren nach Ansprüchen 1 bis 7, dadurch gekennzeichnet, dass in der Stufe der Sulfochlorierung mit einem $Cl_2$/$SO_2$-Gemisch im Molverhältnis von annähernd 1:1 gearbeitet wird.

9. Verfahren nach Ansprüchen 1 bis 8, dadurch gekennzeichnet, dass die Chloraddition an die olefinischen Doppelbindungen bei Temperaturen von 50 bis 70 °C und die Sulfochlorierung bei Temperaturen von 30 bis 50 °C erfolgt.

## Claims

1. A process for the chlorination and sulfochlorination of fatty raw materials which are liquid under reaction conditions, particularly fatty acids and/or esters thereof, by reaction with chlorine and $SO_2$, in which the liquid starting material is circulated through a reaction zone in which it is reacted with chlorine and $SO_2$ introduced into the circuit, characterized in that the chlorine and $SO_2$ are introduced in parallel current with the liquid starting material and, together with the liquid starting material, are passed through an intensive mixer during or before entry into the reaction zone, the quantity of chlorine and $SO_2$ introduced being measured in such a way that the reaction mixture passes through the reaction zone as a substantially homogeneous liquid phase.

2. A process as claimed in Claim 1, characterized in that the starting material used contains olefinically unsaturated fractions and the process is preferably carried out in several stages by initially adding chlorine onto the double bonds, followed by sulfochlorination, the chain-chlorine content of the starting material optionally being increased by a substitution reaction carried out before or after or during sulfochlorination.

3. A process as claimed in Claims 1 and 2, characterized in that esters of $C_8$–$C_{24}$ fatty acids with monohydric and/or polyhydric alcohols are subjected to the process, esters of fatty acids or fatty acid mixtures of natural origin preferably being used.

4. A process as claimed in Claims 1 to 3, characterized in that it is carried out in the absence of viscosityreducing diluents.

5. A process as claimed in Claims 1 to 4, characterized in that the intensive mixing of the gas and liquid phases is produced by static and/or moving mixers known per se built into the liquid circuit, static mixers of which intersecting blades impose a highly turbulent zig-zag flow on the gas-liquid mixture in the mixing zone preferably being used.

6. A process as claimed in Claims 1 to 5, characterized in that the rate of flow of the liquid phase in the static mixer, based on the empty tube, amounts to between 0.1 and 5 m/sec. and more particularly to between 0.25 and 2.5 m/sec., the length-to-diameter ratio of the mixing zone amounting to between 2:1 and 20:1.

7. A process as claimed in Claims 1 to 6, characterized in that the ratio between the quantity of gas introduced and the circulating quantity of liquid for both gaseous reactants amounts to between 0.05 and 500 kg of gas/1000 kg of liquid and, more particularly, to between 0.1 and 100 kg of gas/1000 kg of liquid.

8. A process as claimed in Claims 1 to 7, characterized in that a $Cl_2$/$So_2$ mixture in a molar ratio of approximately 1:1 is used in the sulfochlorination stage.

9. A process as claimed in Claims 1 to 8, characterized in that the addition of chlorine onto the olefinic double bonds is carried out at temperatures in the range from 50 to 70 °C and sulfochlorination at temperatures in the range from 30 to 50 °C.

## Revendications

1. Procédé de chloruration et de sulfochloruration de matières premières grasses liquides dans les conditions de la réaction, en particulier des acides gras et/ou leurs esters, par réaction avec du chlore et du $SO_2$, dans lequel on fait passer la matière ajoutée liquide avec recyclage à travers une zone de réaction dans laquelle elle est mise à réagir avec le chlore et le $SO_2$ alimentés dans le circuit, caractérisé en ce qu'on alimente le chlore et le $SO_2$ en équi-courant avec la matière liquide et, conjointement avec elle, on les fait passer à l'entrée ou avant l'entrée dans la zone de réaction à travers un mélangeur intensif, en l'occurence la quantité du chlore et du $SO_2$ alimentés étant établie en sorte que le mélange de réaction traverse la zone de réaction sous la forme d'une phase liquide pratiquement homogène.

2. Procédé selon la revendication 1, caractérisé en ce qu'on opère avec une matière première contenant des fractions oléfiniquement insaturées, de préférence en plusieurs stades afin que tout d'abord le chlore se fixe sur les doubles liaisons et que la sulfochloruration s'effectue ensuite, cas où si on le désire, dans un stade de réaction qui précède ou qui suit la sulfochloruration, ou pendant la sulfochloruration, la teneur en chlore de la chaîne de la matière utilisée est augmentée par une réaction de substitution.

3. Procédé selon les revendications 1 et 2, caractérisé en ce qu'on soumet au procédé des esters d'acides gras ayant des longueurs de chaîne en $C_8$–$C_{24}$ avec des alcools mono- ou polyvalents, en mettant en jeu de préférence des esters d'acides gras ou mélanges d'acides gras d'origine naturelle.

4. Procédé selon les revendications 1 à 3, caractérisé en ce qu'on opère en l'absence de diluants qui abaissent la viscosité.

5. Procédé selon les revendications 1 à 4, caractérisé en ce qu'on produit le mélange intensif de la phase gazeuse et de la phase liquide à l'aide d'éléments mélangeurs connus en eux-mêmes introduits dans le circuit du liquide et qui sont statiques et/ou mobiles, en opérant de préférence avec des éléments mélangeurs statiques qui, à l'aide d'éléments déflecteurs qui se croisent, engendrent dans l'étendue de la zone de mélange un écoulement à haute turbulence en zigzag du mélange gaz/liquide.

6. Procédé selon les revendications 1 à 5, caractérisé en ce qu'on opère à des vitesses de passage de la phase liquide dans la zone de mélange statique – qui se rapportent au tube vide – de 0,1 à 5 m/seconde, en particulier de l'ordre de 0,25 à 2,5 m/seconde, le rapport longueur/diamètre de l'étendue de mélange se situant de préférence dans l'intervalle de 2:1 à 20:1.

7. Procédé selon les revendications 1 à 6, caractérisé en ce que le rapport de la quantité de gaz ajoutée envers la quantité de liquide en circulation se situe pour les deux réactifs gazeux dans l'intervalle de 0,05 à 500 kg de gaz par 1000 kg de liquide, en particulier dans l'intervalle de 0,1 à 100 kg de gaz/1000 kg de liquide.

8. Procédé selon les revendications 1 à 7, caractérisé en ce que dans le stade de la sulfochloruration on opère avec un mélange $Cl_2/SO_2$ dans le rapport molaire avoisinant 1:1.

9. Procédé selon les revendications 1 à 8, caractérisé en ce que l'addition du chlore sur les doubles liaisons oléfiniques a lieu à des températures de 50 à 70 °C et la sulfochloruration à des températures de 30 à 50 °C.

**HCl**

**B**

**W** Kühlwasser

**G**

**P**

**R**

**SO₂   Cl₂**

Fig.1

Fig. 2

1. Schleife      2. Schleife      3. Schleife

Chlor-Addition      1. Stufe      2. Stufe

0160909